# EUROPÄISCHE PATENTSCHRIFT

(11) **EP 2 593 421 B1**
(45) Veröffentlichungstag und Bekanntmachung des Hinweises auf die Patenterteilung: **15.10.2014**
(21) Anmeldenummer: 11730574.8
(22) Anmeldetag: 29.06.2011
(51) Int. Cl.: C07C 67/60, C07C 69/28

(54) **VERFAHREN ZUR NACHBEHANDLUNG VON POLYOLESTERN**
METHOD FOR AFTERTREATING POLYOLESTERS
PROCÉDÉ POUR LE POST-TRAITEMENT DE POLYOLESTERS

(30) Priorität: 17.07.2010 DE 102010027458
(43) Veröffentlichungstag der Anmeldung: 22.05.2013
(73) Patentinhaber: OXEA GmbH, 46147 Oberhausen (DE)
(72) Erfinder: FREY, Guido D., 64560 Riedstadt (DE); SCHULTZ, Heyko Jürgen, 46236 Bottrop (DE); STRUTZ, Heinz, 47445 Moers (DE)
(86) Internationale Anmeldenummer: PCT/EP2011/003206
(87) Internationale Veröffentlichungsnummer: WO 2012/019670

(56) Entgegenhaltungen:
- EP-A2- 2 308 821
- EP-A2- 2 308 822
- DD-A- 57 596
- DE-A1- 2 729 627
- DE-A1- 10 121 866
- DE-C1- 19 741 913

## Beschreibung

Die Erfindung betrifft ein Verfahren zur Nachbehandlung von Polyolestern, hergestellt durch Umsetzung von linearen oder verzweigten aliphatischen Monocarbonsäuren mit 3 bis 20 Kohlenstoffatomen mit Polyolen in Gegenwart eines Adsorbens und in Gegenwart von metallhaltigen Verbindungen als Katalysatoren, durch Nachbehandlung mit oxidierend oder reduzierend wirkenden Verbindungen und unmittelbar anschließend mit Wasserdampf.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität gezielt einstellen, und den chemischen Eigenschaften, wie der Hydrolyseresistenz oder der Stabilität gegenüber einem oxidativen Abbau Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.

Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylol-propan, Pentaerythrit, 2,2,4-Trimethylpentan-1,3-diol, Glycerin oder 3(4),8(9)-Dihydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Um Weichmachern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen.

Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel. Ethylenoxid ist eine sehr reaktive chemische Substanz. Es kann explosionsartig polymerisieren und bildet mit Luft in sehr weiten Mischungsbereichen explosive Gemische. Ethylenoxid reizt die Augen und Atemwege, führt zu Verätzungen, zu Leber- und Nierenschäden und ist karzinogen. Seine Handhabung erfordert daher umfangreiche Sicherheitsmaßnahmen. Überdies ist auf peinliche Sauberkeit von Lagervorrichtungen und Reaktionsapparaten zu achten, um die Bildung unerwünschter Verunreinigungen durch Nebenreaktionen des Ethylenoxids mit Fremdstoffen auszuschließen. Schließlich ist die Reaktion mit Ethylenoxid nicht sehr selektiv, denn sie führt zu Gemischen von Verbindungen unterschiedlicher Kettenlänge.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d. h. hohe Ausbeuten erzielt werden.

Umfassende Angaben zur Herstellung von Estern mehrwertiger Alkohole, darunter auch Estern aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Zur Entfernung des Reaktionswassers verwendet man Kohlendioxid. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff.

Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich. Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen wird beschrieben. Nach der Lehre von US 2,628,249 lassen sich Farbprobleme bei der Katalyse mit Schwefelsäure oder Sulfonsäure mildern, wenn in Gegenwart von Aktivkohle gearbeitet wird.

Als weitere metallhaltige Katalysatoren werden auch Titan-, Zirconium- oder Zinnalkoholate, -carboxylate oder -chelate zur Herstellung von Polyolestern, beispielsweise gemäß US 5,324,853 A1, eingesetzt. Solche Metallkatalysatoren kann man als Hochtemperaturkatalysatoren ansehen, denn sie erreichen ihre volle Aktivität erst bei hohen Veresterungstemperaturen, im Allgemeinen oberhalb von 180°C. Sie werden häufig nicht zu Beginn der Veresterungsreaktion zugesetzt sondern nachdem das Reaktionsgemisch bereits aufgeheizt wurde und zum Teil unter Wasserabspaltung reagiert hat. Trotz der im Vergleich zur klassischen Schwefelsäurekatalyse erforderlichen höheren Reaktionstemperaturen und längeren Reaktionszeiten werden bei der Katalyse mit solchen metallhaltigen Verbindungen Rohester mit einer vergleichsweise geringen Farbzahl erhalten. Gängige Veresterungskatalysatoren sind beispielsweise Tetra(isopropyl)orthotitanat, Tetra(butyl)orthotitanat, Tetra(butyl)zirconat oder Zinn(II)-2-ethylhexanoat.

Bei der katalytischen Veresterungsreaktion von Polyolen mit Carbonsäuren wird, bezogen auf die im Unterschuss vorliegende Komponente, innerhalb einer vergleichsweise kurzen Zeit ein hoher Umsatz erzielt, doch für den Restumsatz zu den gewünschten Polyolestern muss noch eine verhältnismäßig lange Reaktionsdauer in Kauf genommen werden. Zwar erhält man dann einen Polyolester mit einem akzeptablen Restgehalt an teilveresterten Produkten, ausgedrückt durch die Hydroxylzahl in mg KOH/g (gemäß DIN 53240) oder durch den gaschromatographisch ermittelten Gehalt an teilveresterten Produkten, doch lange Reaktionszeiten sind wirtschaftlich nachteilig, da sie die Leistung der technischen Produktionsanlage begrenzen. Um auch den Restumsatz zu beschleunigen, schlägt US 5,324,853 A1 eine intensive Durchmischung des Reaktionsansatzes vor. Nach beendeter Veresterungsreaktion ist eine ausreichende Abtrennung des Metallkatalysators sicherzustellen, da Metallspuren in den aufgereinigten Polyolestern ihre Anwendung als Weichmacher oder Schmiermittel beeinträchtigen können, indem beispielsweise die elektrische Leitfähigkeit oder die Stabilität gegenüber Luftsauerstoff beeinflusst wird. Gemäß der Arbeitsweise aus US 5,324,853 A1 versetzt man das rohe Veresterungsgemisch mit einer wässrigen Sodalösung und gegebenenfalls mit Aktivkohle. Durch diese Arbeitsweise werden die Metallverbindungen zu unlöslichen Feststoffen hydrolysiert und können vor der weiteren Aufarbeitung der rohen Esterverbindung abfiltriert werden. Nach US 4,304,925 A1 wird das rohe Veresterungsprodukt vor Alkalizugabe zunächst mit Wasser versetzt und in der Wärme behandelt. Dadurch werden die hydrolysierten Metallverbindungen in gut filtrierbare Niederschläge überführt.

Aus US 2, 628, 249 A ist die Veresterung von Etherpolyolen mit aliphatischen Monocarbonsäuren bekannt. Farbprobleme bei der Katalyse mit Schwefelsäure oder Sulfonsäuren lassen sich mildern, wenn die Veresterung in Gegenwart von Aktivkohle durchgeführt wird.

Der Stand der Technik zur Herstellung von Polyolestern unter Metallkatalyse erfordert entweder eine besondere Reaktorauslegung, um die Veresterungsreaktion in einer wirtschaftlich vertretbaren Zeit zu vervollständigen, oder eine zusätzliche Behandlung mit Wasser in der Wärme, um den metallhaltigen Katalysator nach beendeter Veresterungsreaktion unter Bildung gut abfiltrierbarer Hydrolyseprodukte möglichst vollständig zu entfernen.

Obwohl bei der Verwendung metallhaltiger Katalysatoren im Allgemeinen Polyolester mit befriedigender Farbzahl erhalten werden, fallen bei der industriellen Fertigung gelegentlich auch Produkte an, die bezüglich Farbzahl und Säurezahl nicht den Spezifikationswerten entsprechen. Während das Verfahren gemäß DE 10 2009 048 775 A1 die Gewinnung von Polyolestern in hoher Qualität auf einfache Weise ermöglicht, ist es wünschenswert ein Verfahren bereitzustellen, bei dem durch eine einfache Nachbehandlung spezifikationsgerechter Polyolester erhalten wird, falls die nach dem Herstellprozess gemäß DE 10 2009 048 775 A1 erhaltenen Polyolester nicht die geforderte Spezifikation aufweisen sollten, beispielsweise infolge einer während der industriellen Fertigung auftretenden Störung.

Aus EP 2 308 821 A2 ist ein Verfahren zur Farbaufhellung von Polyolestern bekannt, wobei das Reaktionsgemisch im Zuge der Aufarbeitung nach Abtrennung unumgesetzten Ausgangsverbindungen mit Ozon oder ozonhaltigen Gasen behandelt wird und unmittelbar anschließend ohne weitere Zwischenschritte eine Wasserdampfbehandlung erfolgt. Die Aufarbeitung des Reaktionsgemisches wird ohne Adsorbentien durchgeführt. EP 2 308 822 A2 behandelt ein analoges Verfahren zur Farbaufhellung von Polyolestern unter Einsatz von peroxidischen Verbindungen.

Auch nach DE 27 29 627 A1 ist die Behandlung von Carbonsäureestern mit Ozon bekannt. Das Reaktionsgemisch wird nach der Ozonbehandlung mit einer wässrigen Alkalilösung neutralisiert und mit Wasser gewaschen. Flüchtige Bestandteile werden anschließend bei erhöhter Temperatur und unter Vakuum oder bei Normaldruck ausgetrieben. Nach dem Verfahren aus DD 57 596 A werden aromatische Dicarbonsäureester zur Farbaufhellung mit einer wässrigen Wasserstoffperoxidlösung in Gegenwart von Alkali versetzt und anschließend einer Wasserdampfbehandlung unterzogen.

DE 101 21 866 A1 offenbart ein Umesterungsverfahren zur Herstellung von Fettsäurepolyolestern, das in Gegenwart von Reduktionsmitteln und Alkalibasen durchgeführt wird. An die Umesterungsreaktion kann sich eine Bleiche mit Wasserstoffperoxid anschließen. DE 197 41 913 C1 schlägt vor, ein Reaktionsprodukt, das durch Veresterung von Fettsäuren mit Alkoholen unter Sn+4-Katalyse erhalten wird, im Zuge der Aufarbeitung mit einem kombinierten Reduktions- und Fällungsmittel zu versetzen. Dadurch werden schwerlösliche Sn+2-Verbindungen gebildet.

Es bestand daher die Aufgabe ein Verfahren bereitzustellen, bei dem durch eine einfache Nachbehandlung die Qualität bereits unter Metallkatalyse hergestellter und aufgearbeiteter Polyolester derart verbessert werden kann, dass spezifikationsgerechte Polyolester erhalten werden, die möglichst vielseitig angewendet werden können.

Die Erfindung besteht daher in einem Verfahren zur Nachbehandlung von Polyolestern, hergestellt durch Umsetzung von Polyolen der allgemeinen Formel

H-(-O-[-CR¹R²-]ₘ₋)ₒ-OH

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen in Gegenwart eines Adsorbens und in Gegenwart von metallhaltigen Verbindungen ausgewählt aus der Gruppe von titan-, zirkonium-, zinn-, zink-, eisen- und aluminiumhaltigen Verbindungen als Katalysator unter Entfernung des gebildeten Wassers und anschließende Wasserdampfbehandlung, dadurch gekennzeichnet, dass der erhaltene Polyolester zunächst mit einer oxidierend oder reduzierend wirkenden Verbindung und unmittelbar anschließend mit Wasserdampf bei einer Temperatur von 150 bis 250°C und über einen Zeitraum von 0,5 bis 5 Stunden nachbehandelt wird.

Die Reaktion zwischen den Ausgangsverbindungen Polyol und der aliphatischen Monocarbonsäure setzt in Abhängigkeit von den Einsatzmaterialien im Bereich von etwa 120 bis 180°C ein und kann anschließend auf unterschiedlich ausgestaltete Weise zu Ende geführt werden.

Nach einer Ausgestaltung der Veresterungsstufe wird zunächst ausgehend von Raumtemperatur auf eine Temperatur bis auf maximal 280°C, vorzugsweise bis auf 250°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zwei- oder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 250°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend von Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die Anlagenausstattung festgelegt. Ausgehend von Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Die jeweiligen Reaktionsbedingungen, wie Temperatur, Reaktionszeit, anzulegender Druck oder einzusetzender Katalysator sind auf den jeweiligen Polyolester zuzuschneiden, um bei einer ausreichenden Reaktionsgeschwindigkeit die Bildung farbgebender Komponenten in den Hintergrund zu drängen und Abbaureaktionen des Polyolesters möglichst zu vermeiden. Bei Polyolestern auf Basis von Etherdiolen, wie zum Beispiel Triethylenglykol oder Tetraethylenglykol, kann ein verstärkter Abbau des Ethergerüstes einsetzen, wenn die Reaktionsbedingungen, wie Temperatur, Reaktionszeit und Katalysatorart und -menge, nicht gezielt auf den jeweiligen Polyolester eingestellt werden.

Die Veresterung kann mit stöchiometrischen Mengen Polyol und aliphatischer Monocarbonsäure vorgenommen werden. Vorzugsweise lässt man das Polyol jedoch mit überschüssiger Monocarbonsäure reagieren, die im Allgemeinen die leichter siedende Komponente ist und die bei der nachfolgenden Aufarbeitung des Rohesters auf einfache Weise destillativ abgetrennt werden kann. Die aliphatische Monocarbonsäure wird in einem 10 bis 50%-igen molaren, vorzugsweise in einem 20 bis 40 %-igen molaren Überschuss je Mol zu veresternder Hydroxylgruppe des Polyols eingesetzt.
Das gebildete Reaktionswasser wird im Laufe der Veresterungsreaktion zusammen mit der überschüssigen Monocarbonsäure aus dem Reaktionsgefäß abdestilliert und in einen nachgeschalteten Phasentrenner geleitet, in dem sich Monocarbonsäure und Wasser je nach ihren Löslichkeitseigenschaften auftrennen. Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach dem Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion indem man den Reaktionsansatz abkühlen lässt.

Als Katalysatoren für die Veresterung des Polyols mit der Monocarbonsäure verwendet man metallhaltige Verbindungen, ausgewählt aus der Gruppe von titan-, zirkonium-, zinn-, zink-, eisen- und aluminiumhaltigen Verbindungen. Geeignete Verbindungen sind beispielsweise Zinn(II)-oxid, Zinn(IV)oxid, Zinn-Carboxylate, wie Zinn(II)-2-ethylhexanoat, Zinn(II)-oxalat, Zinn(II)-acetat oder Zinn(IV)-acetat, Zinn(IV)-alkoholate, wie Tetra(methyl)stannat, Tetra(ethyl)stannat, Tetra(propyl)stannat, Tetra(iso-propyl)stannat oder Tetra(isobutyl)stannat, oder Organozinnverbindungen, wie Butylzinnmaleat oder Dibutylzinndilaurat.

Zu den geeigneten Titanverbindungen zählen Alkoholate, wie Tetra(methyl)orthotitanat, Tetra(ethyl)orthotitanat, Tetra(propyl)orthotitanat, Tetra(iso-propyl)orthotitanat, Tetra(butyl)orthotitanat, Tetra(iso-butyl)onthotitanat, Tetra(pentyl)orthotitanat oder Tetra(2-ethylhexyl)orthotitanat; Acylate, wie Hydroxytitanacetat, Hydroxytitanbutyrat oder Hydroxytitanpentanoat oder Chelate, wie Tetraethylenglykoltitanat oder Tetrapropylenglykoltitanat. Auch die entsprechenden Zirkoniumverbindungen können mit Erfolg eingesetzt werden, wie Tetra(methyl)orthozirkonat, Tetra(ethyl)orthozirkonat, Zirkoniumcarbonat, Tetra(propyl)orthozirkonat, Tetra(iso-propyl)orthozirkonat, Zirkoniumhydroxid, Tetra(butyl)orthozirkonat, Tetra(iso-butyl)orthozirkonat, Tetra(pentyl)orthozirkonat oder Tetra(2-ethylhexyl)orthozirkonat.

Ebenfalls geeignet sind Aluminiumoxid, Aluminiumhydroxid, Aluminiumcarboxylate, wie Aluminiumacetat oder Aluminiumstearat, oder Aluminiumalkoholate wie Aluminiumtributylat, Aluminium-tri-sec.-butylat, Aluminium-tri-tert.-butylat oder Aluminium-tri-isopropylat.

Auch Zinkoxid, Zinksulfat und Zinkcarboxylate wie Zinkacetat Dihydrat oder Zinkstearat, und Eisen(II)acetat oder Eisen(III)hydroxid-oxid können als Katalysatoren eingesetzt werden.

Es ist ebenfalls möglich, die entsprechenden Metalle in fein verteilter Form einzusetzen, wobei sich die metallhaltige, katalytisch aktive Verbindung zunächst im Reaktionsgemisch bildet.

Der Katalysator kann dem Reaktionsgemisch bereits zu Beginn zugesetzt werden oder erst nachträglich unter Beachtung von Sicherheitsmaßnahmen bei erhöhter Temperatur, wenn beispielsweise die Abtrennung des Reaktionswassers eingesetzt hat.

Die Menge des zugesetzten Veresterungskatalysators beträgt 1 x 10⁻⁵ bis 20 mol%, vorzugsweise 0,01 bis 5 mol-%, insbesondere 0,01 bis 2 mol%, bezogen auf die im Unterschuss zugesetzte Ausgangsverbindung, zweckmäßigerweise bezogen auf Polyol. Bei höheren Katalysatormengen ist mit Spaltungsreaktionen der Polyolester zu rechnen.

Bei der Herstellung von Polyolestern auf Basis von Etherdiolen, wie zum Beispiel Triethylenglykol oder Tetraethylenglykol, ist bei dem Einsatz hoher Katalysatorkonzentrationen gegen Reaktionsende und in der Phase des Umsatzes letzter Reste an freien Hydroxylgruppen eine verstärkte Spaltung der Etherkette zu befürchten, so dass in diesem Falle die Reaktionstemperatur oder der anzulegende Druck anzupassen sind. Je höher die gewählte Katalysatorkonzentration ist, desto niedriger ist im Allgemeinen die Reaktionstemperatur oder der anzulegende Druck zu wählen und es ist nach einem optimierten Temperatur- und Druckprofil zu arbeiten. Bei zu niedrigen Katalysatorkonzentrationen wird die Veresterungsgeschwindigkeit so gering, dass kein akzeptabler Umsatz in einer vertretbaren Reaktionszeit beobachtet wird.

Die Zugabe des Veresterungskatalysators kann in flüssiger oder fester Form erfolgen. Feste Katalysatoren, beispielsweise Zinn(II)oxid, Zinkoxid oder Eisen(III)hydroxid-oxid werden nach beendeter Veresterungsreaktion abfiltriert, bevor der rohe Polyolester der weiteren Aufarbeitung unterzogen wird. Werden die Veresterungskatalysatoren als flüssige Verbindungen zugesetzt, beispielsweise Tetra(iso-propyl)orthotitanat oder Tetra(butyl)orthotitanat, die nach beendeter Veresterungsreaktion im Reaktionsgemisch noch gelöst vorliegen, so werden diese Verbindungen im Laufe des Aufarbeitungsverfahrens bei der Wasserdampfbehandlung in unlösliche, gut abfiltrierbare Niederschläge überführt.

Die Veresterung erfolgt in Gegenwart eines Adsorbens. Man verwendet dabei poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone oder Carbonate, oder Aktivkohle. Besonders bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Die Menge des Adsorbens kann weitgehend frei und somit den individuellen Erfordernissen entsprechend eingestellt werden. Bezogen auf 100 Gewichtsteile des flüssigen Reaktionsansatzes bewährt es sich 0,1 bis 5, vorzugsweise 0,1 bis 1,5 Gewichtsteile des Adsorbens einzusetzen.

Wegen der eingangs beschriebenen Qualitätskriterien für Polyolester sind die Verfahrensschritte bei der Veresterungsstufe unter Entfernung des Reaktionswassers und bei der Aufarbeitung des Rohesters sehr wesentliche Prozessmerkmale, denn die Abstimmung dieser Verfahrensschritte beeinflusst in wesentlichem Maße die sensorischen und optischen Eigenschaften der Endprodukte. Durch eine optimierte Prozessführung werden Polyolester auf Basis von Etherdiolen, beispielsweise Triethylenglykol oder Tetraethylenglykol, mit hoher Reinheit sowie geringer Farbzahl und hoher Farbstabilität gewonnen. Die Struktur der Ausgangsstoffe, der mehrwertigen Alkohole und der aliphatischen Monocarbonsäuren, ist dagegen für die mechanischen und thermischen Eigenschaften der mit den Polyolestern weichgestellten Kunststoffmassen maßgebend und beeinflusst die Hydrolyse- und Oxidationsstabilität von Schmiermitteln.

Das nach Beendigung der Umsetzung anfallende Reaktionsgemisch enthält neben dem Polyolester als erwünschtem Reaktionsprodukt gegebenenfalls nicht umgesetzte Ausgangsstoffe, insbesondere noch überschüssige aliphatische Monocarbonsäure, sofern mit einem Monocarbonsäureüberschuss gearbeitet wird. üblicherweise destilliert man zunächst unumgesetzte und im Überschuss vorliegende Ausgangsverbindungen ab, zweckmäßigerweise unter Anlegen eines verminderten Drucks.
Anschließend wird der Rohester einer Behandlung mit Wasserdampf unterzogen, die beispielsweise in einfacher Form durch Einleiten von Wasserdampf in das Rohprodukt erfolgen kann. Ein Vorteil der Wasserdampfbehandlung ist, dass in ihrem Verlauf noch vorhandener Katalysator zerstört und in gut abfiltrierbare Hydrolyseproduke überführt wird. Da die Veresterungsreaktion in Gegenwart eines Adsorbens durchgeführt wird, erleichtert das bereits anwesende Adsorbens die Abscheidung der Katalysatorfolgeprodukte. Die Anwesenheit eines Adsorbens während der Wasserdampfbehandlung wirkt sich ebenfalls vorteilhaft auf die Farbe und auf die Farbstabilität des Polyolesters aus. Es ist aber auch möglich, nach beendeter Veresterungsreaktion und Abtrennung überschüssiger Ausgangsverbindungen, also vor Durchführung der Wasserdampfdestillation, das Adsorbens abzufiltrieren.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung erfolgt bei Temperaturen von 150 bis 250°C, vorzugsweise von 150 bis 220°C und insbesondere von 170 bis 200°C und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um den Rohester auf die erforderliche Temperatur für die Wasserdampfbehandlung zu erhitzen.

Die Dauer der Wasserdampfbehandlung lässt sich durch Routineversuche ermitteln und sie wird über einen Zeitraum von 0,5 bis 5 Stunden durchgeführt. Eine zu lange Wasserdampfbehandlung führt zu einer unerwünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl beispielsweise bestimmt nach DIN EN ISO 3682/ASTM D 1613 zeigt. Bei einer zu kurzen Behandlungsdauer ist die Restsäure- und Wasserentfernung nicht ausreichend wirksam und der gewünschte Polyolester weist noch eine zu hohe unerwünschte Säurezahl und einen zu hohen Wassergehalt auf. Auch wird bei zu kurzer Behandlungsdauer nur ein geringer vorteilhafter Effekt auf die Farbzahl des Polyolesters beobachtet.

Auch die Bedingungen der Wasserdampfbehandlung wie Temperatur, Dauer und anzulegender Druck sind gezielt auf den jeweiligen Polyolester einzustellen, um ein optimales Ergebnis in Bezug auf die Farbzahl der Polyolester zu erzielen und um Restgehalte an Ausgangsverbindungen, Wasser und Katalysatorspuren möglichst zu minimieren und gleichzeitig Abbaureaktionen zu unterdrücken. Insbesondere bei der Anwendung höherer Katalysatormengen sind bei der Herstellung von Polyolestern auf Basis von Etherdiolen, wie zum Beispiel Triethylenglykol oder Tetraethylenglykol, die Bedingungen bei der Wasserdampfbehandlung auf den jeweiligen Polyolestern genau zuzuschneiden, um den unerwünschten Abbau der Etherkette zu unterbinden.

Gegebenenfalls erfolgt nach der Wasserdampfbehandlung die Zugabe eines festen, alkalisch reagierenden Stoffes, beispielsweise basisches Siliziumdioxid, basisches Aluminiumoxid oder Natriumcarbonat, Natriumhydrogencarbonat, Calciumcarbonat, oder Natriumhydroxid in fester Form sowie basisch reagierende Mineralien, um die Neutralisationszahl des Polyolesters weiter zu verringern.

An die Wasserdampfbehandlung schließt sich, gegebenenfalls nach Filtration des Adsorbens, der gegebenenfalls zugesetzten festen, alkalisch reagierenden Stoffe und weiterer angefallener Feststoffe, die Trocknung des Polyolesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen wie Temperatur, Druck und Dauer lassen sich durch einfache Vorversuche ermitteln. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa und insbesondere 1 bis 20 hPa. Darauf wird der Rohester, falls noch nicht erfolgt, filtriert, um ihn von den Feststoffen, den gegebenenfalls zugesetzten, festen alkalisch reagierenden Stoffen, den Hydrolyseprodukten des Katalysators und dem in der Veresterungsstufe zugesetzten Adsorbens zu befreien. Die Filtration erfolgt in konventionellen Filtrierapparaturen bei normaler Temperatur oder bei Temperaturen bis 120°C. Die Filtration kann durch gängige Filtrierhilfsmittel wie Cellulose, Kieselgel, Kieselgur, Holzmehl, unterstützt werden. Ihr Einsatz ist jedoch auf Ausnahmefälle beschränkt.

Nach Abschluss der Filtration werden hellfarbige Polyolester erhalten, die im Allgemeinen auch den übrigen Spezifikationen, wie Wassergehalt, Restsäuregehalt, Restgehalt an Katalysatorbestandteilen und Restgehalt an Monoester genügen.

Die nach dem erfindungsgemäßen Verfahren nachzubehandelnden Polyolester basieren auf mehrwertigen Alkoholen entsprechend der allgemeinen Formel

H-(-O-[-CR¹R²-]ₘ-)ₒ-OH

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Als Polyole, die zu Polyolestern umgesetzt werden können, eignen sich beispielsweise Di-Trimethylolpropan, Di-Pentaerythrit und die Oligomeren von Ethylenglykol und 1,2-Propylenglykol, insbesondere die Etherdiole Di-, Tri- und Tetraethylenglykol oder Dipropylenglykol, Tripropylenglykol oder Tetrapropylenglykol. Ethylen- und Propylenglykole sind industriell produzierte Chemikalien. Basissubstanz zu ihrer Herstellung ist Ethylenoxid und Propylenoxid, aus dem man 1,2-Ethylenglykol und 1,2-Propylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von Polyolestern setzt man lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher oder Schmiermittel, auch ungesättigte Carbonsäuren als Reaktionskomponente zur Estersynthese verwendet werden. Beispiele für Monocarbonsäuren als Bausteine von Polyolestern sind Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methyl-hexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecancarbonsäure. Besondere Bedeutung besitzt die Herstellung von Polyolestern der oligomeren Ethylenglykole sowie der oligomeren Propylenglykole mit C₄- bis C₁₃- bzw. C₅- bis C₁₀-Monocarbonsäuren sowie zur Herstellung von Polyolestern auf Basis von Di-Trimethylolpropan.

Die Polyolester des Ethylenglykols sowie seine Oligomeren eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Es lassen sich auf einfache Weise Polyolester mit ausgezeichneten Farbeigenschaften herstellen, die auch weiteren Qualitätsansprüchen, wie geringem Geruch oder einer geringen Säurezahl genügen. Besonders Verfahren zur Herstellung von Triethylenglykol-di-2-ethylhexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester) haben Bedeutung erlangt.

Die Veresterung der Ausgangsverbindungen kann kontinuierlich oder absatzweise in den für die chemische Technik typischen Reaktionsapparaten durchgeführt werden. Bewährt haben sich Rührkessel oder Reaktionsrohre, wobei die absatzweise Reaktionsführung die bevorzugte ist.

Obschon das Verfahren gemäß DE 10 2009 048 775 A1 qualitativ hochwertige und spezifikationsgerechte Polyolester liefert, kann es bei Störungen in der industriellen Fertigung zur Produktion von Polyolestern kommen, die hinsichtlich der Farbzahl nicht den Spezifikationswerten genügen.

Nach der erfindungsgemäßen Arbeitsweise können derartige Produktionschargen an Polyolestern auf einfache Weise einer Nachbehandlung unterzogen werden, um die Farbqualität zu verbessern. Hierzu werden die erhaltenen Polyolester zunächst mit einer oxidierend oder reduzierend wirkenden Verbindung behandelt.

Als oxidierend wirkende Verbindung eignen sich peroxidische Verbindungen wie Wasserstoffperoxid, organische Percarbonsäuren, wie Peressigsäure oder Perpropionsäure, organische Hydroperoxide, wie Cumolhydroperoxid oder tert.-Butylhydroperoxid, Alkali- oder Erdalkalimetallperborate, Alkali- oder Erdalkalimetallpercarbonate, Alkali- oder Erdalkalimetallperoxodisulfate oder Alkali- oder Erdalkalimetallperoxophosphate.

Besonders geeignet sind wässrige Wasserstoffperoxidlösungen, flüssige organische Percarbonsäuren oder organische Hydroperoxide, die auf einfache Weise destillativ abgetrennt werden können. Die Anwendung der salzartigen, peroxidischen Alkali- oder Erdalkalimetallverbindungen, entweder in fester Form oder als wässrige Lösung, ist nicht ausgeschlossen aber auf wenige Ausnahmefälle beschränkt, da sie und ihre Umsetzungsprodukte als Feststoffe vorliegen oder im Zuge der Nachbehandlung des Polyolesters sich niederschlagen und durch einen zusätzlichen Filtrationsschritt abgetrennt werden müssen.

Insbesondere geeignet für die Nachbehandlung des bereits aufgearbeiteten Polyolesters ist Wasserstoffperoxid in Form einer wässrigen Lösung mit einem Gehalt an Wasserstoffperoxid von mehr als 10 Gew.-%, vorzugsweise von 30 bis 50 Gew.-%. Wasserstoffperoxidlösungen mit einem geringeren Wirkungsgehalt sind wegen des Eintrags einer zu hohen Menge an Wasser, das anschließende wieder entfernt werden muss, nicht zu empfehlen. Bei zu hohen Wasserstoffperoxidkonzentrationen sind aufwendige und kostspielige Sicherheitsvorkehrungen bei der Handhabung zu beachten.

Man setzt dem zu behandelnden Polyolester die peroxidische Verbindung in einer solchen Menge zu, dass ihr Wirkgehalt in dem gesamten Gemisch von 0,03 bis 1,0 Gew.-%, vorzugsweise von 0,08 bis 0,3 Gew.-%, beträgt. Bei zu geringen Wirkkonzentrationen reicht die Entfärbekraft nicht mehr aus, um hellfarbige Polyolester mit ausreichender Qualität zu erhalten. Bei zu hohen Wirkkonzentrationen ist mit unkontrollierten Abbaureaktionen der Esterverbindungen zu rechnen.

Die Behandlung mit peroxidischen Verbindungen erfolgt im Allgemeinen bei erhöhter Temperatur, vorzugsweise bei Temperaturen von 70 bis 160°C, vorzugsweise 100 bis 120°C, wobei auch niedrige Temperaturen, beispielsweise Raumtemperatur oder darunter, nicht ausgeschlossen werden. Die Behandlungsdauer kann über einen weiten Bereich gewählt werden. Sie sollte nicht zu kurz aber auch nicht zu lang sein und kann durch einfache Vorversuche ermittelt werden. Im Allgemeinen beträgt die Behandlungsdauer 0,5 bis 3 Stunden. Bei kürzeren Behandlungsdauern wird kein positiver Einfluss auf die Farbzahl beobachtet, bei zu langen Behandlungszeiten ist auf Grund des anwesenden Wassers und des Oxidationsmittels eine verstärkte Esterspaltung und ein unkontrollierter Abbau des Polyolester-Gerüstes zu befürchten. Ebenfalls wird bei zu langen Behandlungszeiten unnötig Reaktorvolumen belegt.

Die jeweiligen Bedingungen der Behandlung mit der peroxidischen Verbindung sind auf den jeweiligen Polyolester zuzuschneiden, um eine optimale Entfärbung auf der einen Seite zu erzielen aber auf der anderen Seite Abbaureaktionen des Polyolesters möglichst zu vermeiden. Bei Polyolestern auf Basis von Etherdiolen, wie zum Beispiel Triethylenglykol oder Tetraethylenglykol, kann ein verstärkter Abbau des Ethergerüstes einsetzen, wenn die Bedingungen bei der Behandlung mit der peroxidischen Verbindung, wie Temperatur, Einwirkungsdauer und Konzentration nicht gezielt auf den jeweiligen Polyolester eingestellt werden.

In einer weiteren Ausgestaltung kann die erfindungsgemäße Nachbehandlung der bereits aufgearbeiteten Polyolester zur Verbesserung der Farbqualität mit Ozon oder ozonhaltigen Gasen als oxidierend wirkende Verbindung durchgeführt werden.

Für die Nachbehandlung mit Ozon oder ozonhaltigen Gasen wird Ozon in einer Menge von 0,01 bis 5,0 Gramm, vorzugsweise 0,2 bis 0,8 Gramm, pro Liter Polyolester eingesetzt. Höhere Ozonmengen sind aufgrund verstärkt einsetzender Abbaureaktionen des Polyolestergerüstes nicht zu empfehlen. Neben der Verringerung des gaschromatographisch ermittelten Polyolestergehaltes wird bei einem zu hohen Ozoneintrag auch ein Anstieg in der Säure- oder Neutralisationszahl, beispielsweise bestimmt nach DIN EN ISO 3682/ASTM D 1613, sowie eine Zunahme der Peroxidzahl, ausgedrückt in Milliäquivalent Sauerstoff pro Kilogramm Polyolester und beispielsweise bestimmt nach ASTM E 298, beobachtet. Der Gang dieser Kennzahlen kann mit einer verstärkt einsetzenden Säurebildung gedeutet werden, wenn man eine zu hohe Ozonmenge verwendet. Bei zu geringen Ozoneinträgen ist der vorteilhafte Einfluss auf die Farbaufhellung zu gering oder es müssen unverhältnismäßig lange Behandlungsdauern in Kauf genommen werden.

Man verwendet Ozon entweder in reiner Form oder im Gemisch mit Gasen, beispielsweise mit Luft oder Sauerstoff, oder im Gemisch mit inerten Gasen, wie mit Stickstoff, mit Kohlendioxid oder mit den Edelgasen, wie Helium oder Argon. Setzt man für die Behandlung ozonhaltige Gase ein, so beträgt die Ozonkonzentration zweckmäßigerweise 2 bis 200, vorzugsweise 10 bis 100 Gramm Ozon pro m³ Gasgemisch. Vorzugsweise arbeitet man mit einer Mischung von Ozon in Sauerstoff.

Für die Herstellung von Ozon oder ozonhaltigen Gasgemischen stehen kommerziell erhältliche Ozongeneratoren zur Verfügung, beispielsweise Geräte mit der Bezeichnung Ozone Systems SMO-Series, PDO-Series, SMA-Series oder PDA-Series von der Firma ITT Wedeco GmbH.

Die Behandlung mit Ozon oder ozonhaltigen Gasen kann über einen weiten Temperaturbereich erfolgen. Die untere Temperaturgrenze wird durch die Viskositäts- und Kristallisationseigenschaften des Reaktionsmediums bestimmt, das auch noch bei tiefen Temperaturen ausreichend pumpfähig sein sollte. Bei zu hohen Temperaturen ist mit einer verstärkt einsetzenden Zersetzung des Ozons zu rechnen. Beispielsweise kann über einen Temperaturbereich von -30°C bis hin zu einer Temperatur von 130°C gearbeitet werden. Vorzugsweise wendet man Temperaturen von 20 bis 100°C und insbesondere von 30 bis 80°C an. Die Behandlungsdauer mit Ozon kann sich ebenfalls über einen weiten Bereich erstrecken, üblicherweise wendet man das Oxidationsmittel über wenige Minuten bis hin zu mehreren Stunden an, beispielsweise von einer Minute bis zu drei Stunden, vorzugsweise von 20 bis 90 Minuten. Höhere Temperaturen und längere Behandlungsdauern sind aufgrund einer verstärkt eintretenden Zersetzung des Ozons und auch des Polyolesters zu vermeiden. Bezogen auf die Behandlungsdauer sollte der Ozoneintrag 0,1 bis 5,0 vorzugsweise 0,2 bis 0,9 Gramm Ozon je Stunde und Liter Polyolester betragen.

Die jeweiligen Bedingungen der Behandlung mit Ozon oder ozonhaltigen Gasen sind auf den jeweiligen Polyolester zuzuschneiden, um eine optimale Entfärbung auf der einen Seite zu erzielen aber auf der anderen Seite Abbaureaktionen des Polyolesters möglichst zu vermeiden. Bei Polyolestern auf Basis von Etherdiolen, wie zum Beispiel Triethylenglykol oder Tetraethylenglykol, kann ein verstärkter Abbau des Ethergerüstes einsetzen, wenn die Bedingungen bei der Behandlung mit Ozon oder ozonhaltigen Gasen wie Temperatur, Einwirkdauer oder Ozoneintrag nicht gezielt auf den jeweiligen Polyolester eingestellt werden.

Für die erfindungsgemäße Nachbehandlung der Polyolester zur Verbesserung der Farbqualität sind ebenfalls reduzierend wirkende Verbindungen wie Metallhydride, beispielsweise komplexe Alkalimetallborhydride oder Erdalkalimetallborhydride geeignet.

Man arbeitet zweckmäßigerweise mit Feststoffen, die zunächst in dem zu behandelnden Polyolester suspendiert werden und durch Zugabe von Wasser aktiviert werden. Bei Wasserzugabe wird aus den Metallhydriden, beispielsweise aus den komplexen Alkali- oder Erdalkalimetallborhydriden, reduktiv wirkender Wasserstoff in situ freigesetzt. Der Eintrag zu hoher Wassermengen ist zu vermeiden, da das Wasser anschließend wieder entfernt werden muss sowie zu hohe Wassermengen auch eine zu schnelle Desaktivierung der in situ gebildeten aktiven Wasserstoffverbindungen bewirken. Im Allgemeinen verwendet man, bezogen auf 100 Gew.-Teile nachzubehandelnden Polyolester, von 0,1 bis 5,0 Gew.-Teile, vorzugsweise von 0,1 bis 2,0 Gew.-Teile Wasser.

Man setzt dem zu behandelnden Polyolester die reduzierend wirkende Verbindung in einer solchen Menge zu, dass ihr Wirkgehalt in dem gesamten Gemisch aus Polyolester und zugesetztem Wasser von 0,002 bis 0,3 Gew.-%, vorzugsweise von 0,005 bis 0,05 Gew.-%, beträgt. Bei zu geringen Wirkkonzentrationen reicht die Entfärbekraft nicht mehr aus, um hellfarbige Polyolester mit ausreichender Qualität zu erhalten. Bei zu hohen Wirkkonzentrationen ist mit unkontrollierten Abbaureaktionen der Esterverbindungen zu rechnen.

Die Behandlung mit reduzierend wirkenden Verbindungen erfolgt im Allgemeinen bei erhöhter Temperatur, vorzugsweise bei Temperaturen von 70 bis 160°C, vorzugsweise 80 bis 120°C, wobei auch niedrige Temperaturen, beispielsweise Raumtemperatur oder darunter, nicht ausgeschlossen werden. Die Behandlungsdauer kann über einen weiten Bereich gewählt werden. Sie sollte nicht zu kurz aber auch nicht zu lang sein und kann durch einfache Vorversuche ermittelt werden. Im Allgemeinen beträgt die Behandlungsdauer 0,5 bis 3 Stunden. Bei kürzeren Behandlungsdauern wird kein positiver Einfluss auf die Farbzahl beobachtet, bei zu langen Behandlungszeiten ist auf Grund des anwesenden Wassers und der reduzierend wirkenden Verbindung eine verstärkte Esterspaltung und ein unkontrollierter Abbau des Polyolester-Gerüstes zu befürchten. Ebenfalls wird bei zu langen Behandlungszeiten unnötig Reaktorvolumen belegt.

Die jeweiligen Bedingungen der Behandlung mit der reduzierend wirkenden Verbindung sind auf den jeweiligen Polyolester zuzuschneiden, um eine optimale Entfärbung auf der einen Seite zu erzielen aber auf der anderen Seite Abbaureaktionen des Polyolesters möglichst zu vermeiden. Bei Polyolestern auf Basis von Etherdiolen, wie zum Beispiel Triethylenglykol oder Tetraethylenglykol, kann ein verstärkter Abbau des Ethergerüstes einsetzen, wenn die Bedingungen bei der Behandlung mit der reduzierend wirkenden Verbindung, wie Temperatur, Einwirkungsdauer und Konzentration nicht gezielt auf den jeweiligen Polyolester eingestellt werden.

Nach der oxidativen oder reduktiven Nachbehandlung wird der Polyolester ohne weitere Zwischenschritte unmittelbar anschließend einer Behandlung mit Wasserdampf unterzogen, die beispielsweise in einfacher Form durch Einleiten von Wasserdampf erfolgen kann. Ein Vorteil der Wasserdampfbehandlung ist, dass in ihrem Verlauf überschüssige oxidierend oder reduzierend wirkende Verbindungen zerstört werden und das eingetragene Wasser mit dem Wasserdampf entfernt wird.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung wird bei Temperaturen von 150 bis 250°C, vorzugsweise von 150 bis 220°C und insbesondere von 170 bis 200°C, durchgeführt und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um das Gemisch aus Polyolester und zugesetzter oxidierend oder reduzierend wirkenden Verbindung auf die erforderliche Temperatur für die Wasserdampfbehandlung zu erhitzen.

Die Dauer der Wasserdampfbehandlung lässt sich durch Routineversuche ermitteln und sie wird über einen Zeitraum von 0,5 bis 5 Stunden durchgeführt. Eine zu lange Wasserdampfbehandlung führt zu einer unerwünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl beispielsweise bestimmt nach DIN EN ISO 3682/ASTM D 1613 zeigt. Bei einer zu kurzen Behandlungsdauer ist die Zerstörung der überschüssigen oxidierend wirkenden Verbindung nicht vollständig und der gewünschte Polyolester weist im Falle der Nachbehandlung mit einer oxidierend wirkenden Verbindung noch eine zu hohe unerwünschte Peroxidzahl, ausgedrückt in Milliäquivalent Sauerstoff pro Kilogramm Produkt und bestimmt nach ASTM E 298, auf. Auch wird bei zu kurzer Behandlungsdauer nur ein geringer vorteilhafter Effekt auf die Farbzahl des Polyolesters beobachtet.

Wie bei der Behandlung mit der oxidierend oder reduzierend wirkenden Verbindung sind auch bei der sich unmittelbar anschließenden Wasserdampfbehandlung die Bedingungen, wie Temperatur, Druck und Dauer gezielt auf den jeweiligen Polyolester einzustellen, um ein optimales Ergebnis in Bezug auf die Farbzahl des Polyolesters zu erzielen und um Restgehalte an eingetragenem Wasser sowie an Peroxidspuren möglichst zu minimieren und gleichzeitig Abbaureaktionen zu unterdrücken. Bei Polyolestern auf Basis von Etherdiolen, wie zum Beispiel Triethylenglykol oder Tetraethylenglykol, sind die Bedingungen bei der Wasserdampfbehandlung auf den jeweiligen Polyolester genau zuzuschneiden, um den unerwünschten Abbau der Etherkette zu unterbinden.

Im Allgemeinen wird nach der Wasserdampfbehandlung ein spezifikationsgerechter Polyolester erhalten und weitere Nachbehandlungsschritte, wie eine zusätzliche Trocknung sind entbehrlich, jedoch nicht ausgeschlossen.

Arbeitet man mit Metallhydriden oder Alkali- oder Erdalkalimetallborhydriden als reduzierend wirkende Verbindung, erweist sich als zweckmäßig, die Wasserdampfbehandlung in Gegenwart einer alkalisch reagierenden Verbindung durchzuführen, beispielsweise mit Natriumhydroxid oder Natriumcarbonat, entweder in fester Form oder als wässrige Lösung. Bezogen auf ein Äquivalent der vom Metall oder Bor abgeleiteten Säure werden 1,2 bis 1,5 Äquivalente an alkalisch reagierender Verbindung zugesetzt.

Nach der Wasserdampfbehandlung fallen metallhaltige oder borhaltige Feststoffe an. In diesen Fällen schließt sich an die Wasserdampfbehandlung unmittelbar ohne weitere Zwischenschritte die Trocknung des nachbehandelten Polyolesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen, wie Temperatur, Druck und Dauer lassen sich durch einfache Vorversuche ermitteln und sind auf den jeweiligen Polyolester zuzuschneiden. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa, und insbesondere 1 bis 20 hPa. Nach beendeter Trocknung werden abgeschiedene metallhaltige oder borhaltige Feststoffe, die sich während der Wasserdampfbehandlung oder während der Trocknung gebildet haben, abfiltriert.

Durch die erfindungsgemäße Nachbehandlung von Polyolestern können Farbprobleme behoben werden, wenn bei der industriellen Fertigung gemäß dem Verfahren der DE 10 2009 048 775 A1 Produktionschargen anfallen, die hinsichtlich der geforderten Farbzahl außerhalb der Spezifikation liegen.

In den folgenden Beispielen wird das erfindungsgemäße Verfahren näher erläutert, es ist jedoch nicht auf die beschriebene Ausführungsform beschränkt.

### Ausführungsbeispiele (Vergleichsbeispiele)

Für die Versuche zur Farbaufhellung kam katalytisch hergestelltes Triethylenglykol-di-2-ethyl-hexanoat mit einer Farbzahl von 37 Hazen-Einheiten zum Einsatz, das durch Zuhilfenahme eines Titankatalysators durch Veresterung von Triethylenglykol mit einer 2,6 molaren Menge an 2-Ethylhexansäure gewonnen wurde. Die Katalysatorkonzentration betrug 0,05 mol%, bezogen auf Triethylenglykoleinsatz. Der gaschromatographisch ermittelte Gehalt (Gew.-%) an Triethylenglykol-di-2-ethylhexanoat betrug 97,9%, an Triethylenglykol-mono-2-ethylhexanoat 1,0% und der Rest auf 100% betrug 1,1%.

### Beispiel 1 zur Nachbehandlung:

Eine Nachbehandlung zur Farbaufhellung des katalytisch hergestellten Triethylenglykol-di-2-ethylhexanoats mit einer wässrigen Wasserstoffperoxidlösung erfolgte unter folgenden Bedingungen:

| | |
|---|---|
| Konzentration der wässrigen H₂O₂-Lösung | 30 Gew.-% |
| Mengen H₂O₂, absolut bezogen auf das gesamte Reaktionsgemisch | 0,10 Gew.-% |
| Reaktionstemperatur | 120°C |
| Reaktionsdauer | 2 Stunden |

Die unmittelbar anschließende Wasserdampfdestillation wurde unter-folgenden Bedingungen durchgeführt:

| | |
|---|---|
| Arbeitstemperatur der Wasserdampfdestillation | 180°C |
| Behandlungsdauer | 1 Stunde |

Die anschließende Trocknung wurde unter folgenden Bedingungen durchgeführt:

| | |
|---|---|
| Druck | 10 hPa |
| Trocknungstemperatur | 80°C |
| Trocknungsdauer | 1 Stunde |

Nach Abschluss der Aufarbeitung erhielt man einen hellfarbigen Polyolester mit folgenden gaschromatographisch ermittelten Gehalten:

| | |
|---|---|
| Gehalt an Triethylenglykol-di-2-ethylhexanoat | 97,6 Gew.-% |
| Gehalt an Triethylenglykol-mono-2-ethylhexanoat | 1,0 Gew.-% |
| Rest | 1,4 Gew.-% |

und folgenden Kennzahlen:

| | |
|---|---|
| Farbzahl nach Hazen (DIN ISO 6271) | 11 |
| Neutralisationszahl (mg KOH/g, DIN EN ISO 3682/ ASTM D 1613) | 0,06 |
| Wassergehalt (Gew.-%, DIN 51777 Teil 1) | 0,02 |
| Peroxidgehalt (mäq O/kg, ASTM E 298) | 0,9 |

### Beispiel 2 zur Nachbehandlung:

Die Nachbehandlung des Triethylenglykol-di-2-ethylhexanoats wurde mit jeweils 1 Liter Rohprodukt in einem beheizbaren 2 Liter-Vierhalskolben durchgeführt, der mit Rührer, Innenthermometer und Zuleitung mit einer Perlfritte der Porengröße G3 ausgestattet wurde. In dem Ozongenerator Modular 8HC (BHT 964) der Firma ITT Wedeco GmbH wurde ein ozonhaltiger Sauerstoffstrom mit einer Ozonkonzentration von 21 Gramm Ozon pro Kubikmeter Sauerstoff erzeugt, der mit einer Rate von 0,025 m³/Stunde über die Perlfritte durch den Rohester bei einer Temperatur von 70°C über einen Zeitraum von 0,5 Stunden unter intensivem Rühren geleitet wurde.

Für die sich anschließende Wasserdampfdestillation ersetzte man die Ozonzuleitung durch eine Destillationsbrücke mit einer 1 Liter-Vorlage und stattete den 2 Liter-Vierhalskolben mit einem Tauchrohr zur Durchleitung von Wasserdampf aus.

Nach Durchführung der Wasserdampfdestillation gemäß den nachfolgend beschriebenen Bedingungen unterbrach man die Zufuhr von Wasserdampf und man legte zur abschließenden Trocknung über die Destillationsbrücke einen Unterdruck an. Man erhielt als Rückstand einen hellfarbigen, spezifikationsgerechten Polyolester.

Die unmittelbar an die Ozonbehandlung anschließende Wasserdampfdestillation wurde unter folgenden Bedingungen durchgeführt:

| | |
|---|---|
| Arbeitstemperatur der Wasserdampfdestillation | 180°C |
| Behandlungsdauer | 1 Stunde |

Anschließend wurden folgende Trocknungsbedingungen eingestellt:

| | |
|---|---|
| Druck | 10 hPa |
| Trocknungstemperatur | 80°C |
| Trocknungsdauer | 1 Stunde |

Nach Abschluss der Aufarbeitung erhielt man einen hellfarbigen Polyolester mit folgenden gaschromatographisch ermittelten Gehalten:

| | |
|---|---|
| Gehalt an Triethylenglykol-di-2-ethylhexanoat | 97,5 Gew.-% |
| Gehalt an Triethylenglykol-mono-2-ethylhexanoat | 1,0 Gew.-% |
| Rest | 1,5 Gew.-% |

und folgenden Kennzahlen:

| | |
|---|---|
| Farbzahl nach Hazen (DIN ISO 6271) | 12 |
| Neutralisationszahl (mg KOH/g, DIN EN ISO 3682/ ASTM D 1613) | 0,06 |
| Wassergehalt (Gew.-%, DIN 51777 Teil 1) | 0,03 |
| Peroxidgehalt (mäq O/kg, ASTM E 298) | 1,15 |

### Beispiel 3 zur Nachbehandlung:

Eine Nachbehandlung zur Farbaufhellung des katalytisch hergestellten Triethylenglykol-di-2-ethylhexanoats mit Natriumborhydrid erfolgte unter folgenden Bedingungen:

| | |
|---|---|
| Zugesetzte Wassermenge auf 100 Gew.-Teile Polyolester | 1,5 Gew.-Teile |
| Konzentration an NaBH₄ | 150 ppm |
| Reaktionstemperatur | 120°C |
| Reaktionsdauer | 2 Stunden |

Die unmittelbar an die Natriumborhydridbehandlung anschließende Wasserdampfbehandlung erfolgte unter folgenden Bedingungen:

| | |
|---|---|
| Zugabe von festem Natriumcarbonat | 1,2 Äquivalente auf 1 Äquivalent Borsäure |
| Arbeitstemperatur der Wasserdampfdestillation | 180°C |
| Behandlungsdauer | 1 Stunde |

Die anschließende Trocknung wurde unter folgenden Bedingungen durchgeführt:

| | |
|---|---|
| Druck | 10 hPa |
| Trocknungstemperatur | 80°C |
| Trocknungsdauer | 1 Stunde |

Nach Abschluss der Aufarbeitung und Filtration erhielt man einen hellfarbigen Polyolester mit folgenden gaschromatographisch ermittelten Gehalten:

| | |
|---|---|
| Gehalt an Triethylenglykol-di-2-ethylhexanoat | 97,3 Gew.-% |
| Gehalt an Triethylenglykol-mono-2-ethylhexanoat | 1,1 Gew.-% |
| Rest | 1,6 Gew.-% |

und folgenden Kennzahlen:

| | |
|---|---|
| Farbzahl nach Hazen (DIN ISO 6271) | 19 |
| Neutralisationszahl (mg KOH/g, DIN EN ISO 3682/ ASTM D 1613) | 0,08 |
| Wassergehalt (Gew.-%, DIN 51777 Teil 1) | 0,03 |

## Patentansprüche

1. Verfahren zur Nachbehandlung von Polyolestern, hergestellt durch Umsetzung von Polyolen der allgemeinen Formel
H-(-O-[-CR¹R²-]ₘ-)ₒ-OH
in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen in Gegenwart eines Adsorbens und in Gegenwart von metallhaltigen Verbindungen, ausgewählt aus der Gruppe von titan-, zirkonium-, zinn-, zink-, eisen- und aluminiumhaltigen Verbindungen als Katalysator unter Entfernung des gebildeten Wassers und anschließende Wasserdampfbehandlung, **dadurch gekennzeichnet, dass** der erhaltene Polyolester zunächst mit einer oxidierend oder reduzierend wirkenden Verbindung und unmittelbar anschließend mit Wasserdampf bei einer Temperatur von 150 bis 250°C und über einen Zeitraum von 0,5 bis 5 Stunden nachbehandelt wird.

2. Verfahren gemäß Anspruch 1, **dadurch gekennzeichnet, dass** als oxidierend wirkende Verbindung peroxidische Verbindungen verwendet werden.

3. Verfahren gemäß Anspruch 2, **dadurch gekennzeichnet, dass** als peroxidische Verbindungen Wasserstoffperoxid, Peressigsäure, Perpropionsäure, Cumolhydroperoxid, tert.-Butylhydroperoxid, Alkali- oder Erdalkalimetallperborate, Alkali- oder Erdalkalimetallpercarbonate, Alkali- oder Erdalkalimetallperoxodisulfate oder Alkali- oder Erdalkalimetallperoxophosphate verwendet werden.

4. Verfahren gemäß Anspruch 3, **dadurch gekennzeichnet, dass** Wasserstoffperoxid in Form einer wässrigen Lösung eingesetzt wird.

5. Verfahren gemäß Anspruch 1, **dadurch gekennzeichnet, dass** als oxidierend wirkende Verbindung Ozon oder ozonhaltige Gase verwendet werden.

6. Verfahren gemäß Anspruch 5, **dadurch gekennzeichnet, dass** Mischungen aus Ozon und Sauerstoff verwendet werden.

7. Verfahren gemäß Anspruch 1, **dadurch gekennzeichnet, dass** als reduzierend wirkende Verbindung Metallhydride, insbesondere komplexe Alkalimetallborhydride oder Erdalkalimetallborhydride verwendet werden.

8. Verfahren gemäß Anspruch 7, **dadurch gekennzeichnet, dass** während der Verwendung von Metallhydriden Wasser zugesetzt wird und die unmittelbar anschließende Behandlung mit Wasserdampf in Gegenwart einer alkalisch reagierenden Verbindung erfolgt.

9. Verfahren gemäß einem oder mehreren der Ansprüche 1, 7 und 8 **dadurch gekennzeichnet, dass** nach der Wasserdampfbehandlung eine Trocknung und Filtration erfolgt.

10. Verfahren gemäß einem oder mehreren der Ansprüche 1 bis 9, **dadurch gekennzeichnet, dass** man als Polyole Di-Trimethylolpropan, Di-Pentaerythrit, Diethylenglykol, Triethylenglykol, Tetraethylenglykol, Dipropylenglykol, Tripropylenglykol oder Tetrapropylenglykol verwendet.

11. Verfahren gemäß einem oder mehreren der Ansprüche 1 bis 10, **dadurch gekennzeichnet, dass** man als aliphatische Monocarbonsäure Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Tri-methylhexansäure oder 2-Propylheptansäure umsetzt.

12. Verfahren gemäß einem oder mehreren der Ansprüche 1 bis 11 zur Nachbehandlung von Triethylenglykol-di-2-ethylhexanoat, Tetraethylenglykol-di-n-heptanoat, Triethylenglykol-di-2-ethylbutyrat, Triethylenglykol-di-n-heptanoat oder Tetraethylenglykol-di-2-ethyl-hexanoat.

## Claims

1. Process for aftertreatment of polyol esters prepared by reacting polyols of the general formula
H-(-O-[-CR¹R²-]ₘ-)ₒ-OH
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer from 1 to 10, preferably 1 to 8 and especially 1, 2, 3 or 4, is an integer from 2 to 15, preferably 2 to 8 and especially 2, 3, 4 or 5, with linear or branched aliphatic monocarboxylic acids having 3 to 20 carbon atoms in the presence of an adsorbent and in the presence of metal compounds selected from the group of titanium compounds, zirconium compounds, tin compounds, zinc compounds, iron compounds and aluminium compounds as a catalyst while removing the water formed and subsequently treating with steam, **characterized in that** the polyol ester obtained is aftertreated first with an oxidizing or reducing compound and immediately thereafter with steam at a temperature of 150 to 250°C and over a period of 0.5 to 5 hours.

2. Process according to Claim 1, **characterized in that** the oxidizing compounds used are peroxidic compounds.

3. Process according to Claim 2, **characterized in that** the peroxidic compounds used are hydrogen peroxide, peracetic acid, perpropionic acid, cumene hydroperoxide, tert-butyl hydroperoxide, alkali metal or alkaline earth metal perborates, alkali metal or alkaline earth metal percarbonates, alkali metal or alkaline earth metal peroxodisulphates or alkali metal or alkaline earth metal peroxophosphates.

4. Process according to Claim 3, **characterized in that** hydrogen peroxide is used in the form of an aqueous solution.

5. Process according to Claim 1, **characterized in that** the oxidizing compound used is ozone or ozone-containing gases.

6. Process according to Claim 5, **characterized in that** mixtures of ozone and oxygen are used.

7. Process according to Claim 1, **characterized in that** the reducing compounds used are metal hydrides, especially complex alkali metal borohydrides or alkaline earth metal borohydrides.

8. Process according to Claim 7, **characterized in that** water is added during the use of metal hydrides and the immediately subsequent treatment with steam is effected in the presence of an alkaline compound.

9. Process according to one or more of Claims 1, 7 and 8, **characterized in that** the steam treatment is followed by drying and filtration.

10. Process according to one or more of Claims 1 to 9, **characterized in that** the polyols used are ditrimethylolpropane, dipentaerythritol, diethylene glycol, triethylene glycol, tetraethylene glycol, dipropylene glycol, tripropylene glycol or tetrapropylene glycol.

11. Process according to one or more of Claims 1 to 10, **characterized in that** the aliphatic monocarboxylic acid converted is propionic acid, n-butyric acid, isobutyric acid, n-pentanoic acid, 2-methylbutyric acid, 3-methylbutyric acid, 2-methylpentanoic acid, n-hexanoic acid, 2-ethylbutyric acid, n-heptanoic acid, 2-methylhexanoic acid, 2-ethylhexanoic acid, n-nonanoic acid, 2-methyloctanoic acid, isononanoic acid, 3,5,5-trimethylhexanoic acid or 2-propylheptanoic acid.

12. Process according to one or more of Claims 1 to 11 for aftertreatment of triethylene glycol di-2-ethylhexanoate, tetraethylene glycol di-n-heptanoate, triethylene glycol di-2-ethylbutyrate, triethylene glycol di-n-heptanoate or triethylene glycol di-2-ethylhexanoate.

## Revendications

1. Procédé pour le post-traitement d'esters de polyols, préparés par mise en réaction de polyols de formule générale
H-(-O-[-CR¹R²-]ₘ-)ₒ-OH
dans laquelle R¹ et R² représentent indépendamment l'un de l'autre un atome d'hydrogène, un radical alkyle ayant de 1 à 5 atomes de carbone, de préférence méthyle, éthyle ou propyle, ou un radical hydroxyalkyle ayant de 1 à 5 atomes de carbone, de préférence le radical hydroxyméthyle, m représente un nombre entier valant de 1 à 10, de préférence de 1 à 8 et en particulier 1, 2, 3 ou 4, o représente un nombre entier valant de 2 à 15, de préférence de 2 à 8 et en particulier 2, 3, 4 ou 5, avec des acides monocarboxyliques aliphatiques linéaires ou ramifiés ayant de 3 à 20 atomes de carbone, en présence d'un adsorbant et en présence de composés contenant des métaux, choisis dans le groupe des composés contenant du titane, du zirconium, de l'étain, du zinc, du fer et de l'aluminium, en tant que catalyseur, avec élimination de l'eau formée et traitement subséquent à la vapeur d'eau, **caractérisé en ce qu'**on soumet le polyester obtenu à un post-traitement d'abord par un composé à action oxydante ou réductrice et immédiatement ensuite par de la vapeur d'eau à une température de 150 à 250 °C et pendant une durée de 0,5 à 5 heures.

2. Procédé selon la revendication 1, **caractérisé en ce qu'**on utilise comme composé à action oxydante des composés de type peroxyde.

3. Procédé selon la revendication 2, **caractérisé en ce qu'**on utilise comme composés de type peroxyde le peroxyde d'hydrogène, l'acide peracétique, l'acide perpropionique, l'hydroperoxyde de cumène, l'hydroperoxyde de tert-butyle, des perborates de métaux alcalins ou alcalino-terreux, des percarbonates de métaux alcalins ou alcalino-terreux, des peroxodisulfates de métaux alcalins ou alcalino-terreux ou des peroxophosphates de métaux alcalins ou alcalino-terreux.

4. Procédé selon la revendication 3, **caractérisé en ce qu'**on utilise du peroxyde d'hydrogène sous forme d'une solution aqueuse.

5. Procédé selon la revendication 1, **caractérisé en ce qu'**on utilise comme composé à action oxydante l'ozone ou des gaz contenant de l'ozone.

6. Procédé selon la revendication 5, **caractérisé en ce qu'**on utilise des mélanges d'ozone et d'oxygène.

7. Procédé selon la revendication 1, **caractérisé en ce qu'**on utilise comme composé à action réductrice des hydrures métalliques, en particulier des borohydrures de métaux alcalins ou borohydrures de métaux alcalino-terreux complexes.

8. Procédé selon la revendication 7, **caractérisé en ce que** pendant l'utilisation d'hydrures métalliques on ajoute de l'eau et le traitement immédiatement subséquent s'effectue avec de la vapeur d'eau en présence d'un composé à réaction alcaline.

9. Procédé selon une ou plusieurs des revendications 1, 7 et 8, **caractérisé en ce qu'**après le traitement par la vapeur d'eau on effectue un séchage et une filtration.

10. Procédé selon une ou plusieurs des revendications 1 à 9, **caractérisé en ce qu'**on utilise comme polyols le di-triméthylolpropane, le di-pentaérythritol, le diéthylèneglycol, le triéthylèneglycol, le tétraéthylèneglycol, le dipropylèneglycol, le tripropylèneglycol ou le tétrapropylèneglycol.

11. Procédé selon une ou plusieurs des revendications 1 à 10, **caractérisé en ce qu'**on utilise comme acide monocarboxylique aliphatique l'acide propionique, l'acide n-butyrique, l'acide isobutyrique, l'acide n-pentanoïque, l'acide 2-méthylbutyrique, l'acide 3-méthylbutyrique, l'acide 2-méthylpentanoïque, l'acide n-hexanoïque, l'acide 2-éthylbutyrique, l'acide n-heptanoïque, l'acide 2-méthylhexanoïque, l'acide 2-éthylhexanoïque, l'acide n-nonanoïque, l'acide 2-méthyloctanoïque, l'acide isononanoïque, l'acide 3,5,5-triméthylhexanoïque ou l'acide 2-propyl-heptanoïque.

12. Procédé selon une ou plusieurs des revendications 1 à 11, destiné au post-traitement du di-2-éthylhexanoate de triéthylèneglycol, du di-n-heptanoate de tétraéthylèneglycol, du di-2-éthylbutyrate de triéthylèneglycol, du di-n-heptanoate de triéthylèneglycol ou du di-2-éthylhexanoate de tétraéthylèneglycol.
